# EUROPÄISCHE PATENTSCHRIFT

(11) **EP 3 247 416 B1**
(45) Veröffentlichungstag und Bekanntmachung des Hinweises auf die Patenterteilung: **31.08.2022**
(21) Anmeldenummer: 16703441.2
(22) Anmeldetag: 19.01.2016
(51) Int. Cl.: A61L 27/42

(54) **BIOKOMPATIBLES FORMTEIL**
BIOCOMPATIBLE MOLDED PART
PIÈCE MOULÉE BIOCOMPATIBLE

(30) Priorität: 20.01.2015 DE 102015100806
(43) Veröffentlichungstag der Anmeldung: 29.11.2017
(73) Patentinhaber: Alexakis, Antonis, 78337 Öhningen (DE)
(72) Erfinder: Alexakis, Antonis, 78337 Öhningen (DE)
(74) Vertreter: Baumann, Rüdiger Walter
(86) Internationale Anmeldenummer: PCT/EP2016/051042
(87) Internationale Veröffentlichungsnummer: WO 2016/116465

(56) Entgegenhaltungen:
- EP-A2- 2 572 738
- WO-A1-2013/079443
- US-A1- 2009 017 095
- US-A1- 2014 120 149

## Beschreibung

Die Erfindung betrifft ein biokompatibles Formteil zur Unterstützung der Knochenneubildung.

In der Medizin gibt es eine Vielzahl von Anwendungsfällen, in denen es wünschenswert ist, dass Knochenmaterial vom menschlichen oder tierischen Patienten selbst neu gebildet wird. Dies gilt in besonderem Maße in der Zahnmedizin, wenn der Kieferknochen z. B. durch Parodontitis angegriffen und teilweise zerstört ist.

Es ist bekannt, für das gezielte Wachstum der Osteoblasten am menschlichen oder tierischen Kieferknochen Hohlräume in einem Formteil zu bilden. In diesen können Osteoblasten aufwachsen und dabei Knochendefekte füllen bzw. einen Kieferaufbau in Höhe und/oder Breite durchführen. Als nachteilig erweist sich, dass manche dieser Materialien insbesondere bei größeren Läsionen keine ausreichende Stabilität für eine zufriedenstellende Osteogenese bzw. Ossifikation zur Verfügung stellen und zudem zu schnell und ungleichmäßig resorbiert werden. Das Ziel der Auffüllung oder des Aufbaus wird dadurch oftmals nicht erreicht.

US 2014/120149 A1 offenbart ein Implantat mit einem porösen Titanteil zum Einsetzen in einen Knochen. Der zwischen Implantat und Knochen verbleibende, Spalt wird mit einem Füllmaterial aus Gips und Granulatmaterial ausgefüllt. Ein formstabiles, blockförmiges Formteil wird nicht offenbart.

US2009/017095 A1 offenbart ein nicht formstabiles Element zur Auffüllung von Knochenfehlstellen.

WO 2013/079443 A1 zeigt ein schalenartiges Element, das einen Hohlraum definiert, der als Aufwachsraum für Knochenmaterial dienen soll.

EP 2 572 738 A2 offenbart einen komprimierbaren, aus Kollagenmaterial mit einem eingebetteten Knochenersatzmaterial gebildeten Formkörper, der in Wundhöhlen oder Alveolen eingesetzt wird. Der Formkörper ist nicht formstabil oder aus Vollmaterial gebildet.

Andere Materialien werden zwar sehr langsam resorbiert, lassen in den höheren Schichten jedoch kein Osteoblastenwachstum mehr zu, da aufgrund der langen Verweildauer der Barriere kein Nährboden für das Osteoblastenwachstum mehr vorhanden ist.

Aufgabe der vorliegenden Erfindung ist es daher, die Nachteile des Standes der Technik zu überwinden und ein das Osteoblastenwachstum begünstigendes Formteil zur Verfügung zu stellen.

Diese Aufgabe wird gelöst durch ein biokompatibles Formteil gemäß Anspruch 1. Bevorzugte Ausführungsformen der Erfindung sind Gegenstand der abhängigen Ansprüche.

Erfindungsgemäß ist ein biokompatibler Formkörper vorgesehen, der zur Unterstützung der Neubildung eines Kieferknochens oder eines Kieferknochenabschnittes in einem Säugetier, bevorzugt in einem Menschen dient. Das Formteil ist dabei auf dem Kieferknochen auflegbar mäß der Erfindung ist als Vollkörper ausgebildet, in den im Verlauf der Behandlung Gefäßen und Osteoblasten einwachsen. Im Zuge der schrittweisen Resorption des Formteils erfolgt dann eine Knochenbildung. Dieser Vollkörper gibt einerseits eine ausreichende Stabilität für die Neubildung des Kieferknochens während des Osteoblastenwachstums, andererseits ist der Vollkörper derart beschaf-fen, dass dieser von Gefäßen und sich neu bildendem Knochenmaterial vollständig durchwachsen werden kann. Aufgrund der Biokompatibilität wird durch den Vollkörper bzw. das diesen bildende Material die Osteogenese gefördert und gleichzeitig eine Stabilisierung des Kieferknochens erreicht bis der Knochenaufbau abgeschlossen ist.

Während der Verwendung, d.h. im Verlauf der Knochenbildung wird das Formteil bzw. der Vollkörper Stück für Stück resorbiert. Diese Resorption geht einher mit einer entsprechenden Auffüllung der Läsion mit neugebildeten Knochenmaterial bzw. die Ausfüllung mit Osteoblasten, die die Osteogenese initiieren bzw. durchführen.

Überraschend hat sich gezeigt, dass gerade die gewählte Blockform die Ossifikation bzw. Osteogenese vorteilhaft unterstützt, da eine Einwanderung von Osteoblasten von wenigstens zwei Seiten her möglich ist und somit eine vollständigere und gleichmäßigere Durchwachsung stattfinden kann. Es bildet sich eine gleichmäßigere Knochenwulst, die sich insgesamt als für die Stabilität des neugebildeten Knochens förderlich erweist. Es ergeben sich hieraus Vorteile beim Einbau von Implantaten in den neugebildeten Knochen.

Die Erfindung stellt somit ein Formteil zur Verfügung das zunächst einen Raum für das Einwachsen von Gefäßen und die anschließende Ossifkation bildet. Das Formteil dient zum temporären Befüllen oder Überbrücken von Knochendefekten und -läsionen, die allein durch körpereigene Regenerationsfähigkeit nicht behoben werden können. Gleichzeitig kann das Formteil auch als Füllelement bei rekonstruktiven Eingriffen, Knochentumoren oder zur Augmentationen, bspw. vor Insertion dentaler Implantate dienen.

Bis zum Abschluss der Behandlung ist das Formteil vollständig durch neugebildeten Knochensubstanz ersetzt. Die Bestandteile des Formteils werden sukzessive resorbiert und geben somit Raum frei, in dem eine Knochenneubildung erfolgen kann. Bis dieser Raum mit Knochenmaterial gefüllt ist, stabilisiert das sich langsam abbauende Formteil die neu entstehende Knochenstruktur. Das Formteil ist aus einer Zusammensetzung gebildet die aus wenigstens einem strukturgebenden Material und einem Granulat besteht oder dieses umfasst. Die Zusammensetzung bleibt dabei nicht auf diese beiden Komponenten beschränkt sondern kann zusätzlich weitere umfassen. Zur Herstellung des Formteils wird der Zusammensetzung zusätzlich Wasser, bevorzugt destillierte Wasser beigegeben, um die Modellierbarkeit zu gewährleisten. Dieses Wasser wird entweder durch die Zusammensetzung gebunden oder verdunstet im Verlauf der Trocknung des fertiggestellten Formteils.

Das strukturgebende Material ist ausgewählt aus der Gruppe bestehend aus Alabastergips, Hartgips, Superhartgips, oder Mischungen daraus. Alle vorgenannten Materialien können vollständig resorbiert werden und sind aus der Verwendung im kieferchirurgischen Bereich und der Implantat-Medizin bekannt und die dortige Verwendung geeignet. Bevorzugt wird Alabastergips verwendet, der neben einer entsprechenden Resorbierbarkeit eine ausreichende Stabilität des Formteils bzw. Vollkörpers während des Knochenwachstums gewährleistet. Gleichzeitig begünstigt dieses Material das Einwachsen von Gefäßen. Der Begriff Alabastergips ist ein aus der Zahnkunde hinlänglich bekannter Begriff. Bezüglich seiner Härte liegt Alabastergips zwischen dem ebenfalls im zahntechnischen Bereich verwendeten Abdruckgips sowie einem Hartgips. Dieser Gips kann nach Herstellung des Formteils mit einfachen zahnmedizinischen Werkzeugen weiter bearbeitet werden und lässt somit eine Anpassung des Vollkörpers an die Gegebenheiten im Kiefer zu.

Das Formteil ist auch in der Lage sich bildende Blutgerinnsel aufzunehmen und zu stabilisieren. Es erhöhen sich dadurch die Chancen, dass im gesamten Formteil gleichzeitig Gefäße wachsen können. Diese Gefäße dienen zur Nährstoffversorgung der Osteoblasten, sodass es zu einer Ossifikation bzw. Osteogenese kommen kann, in deren Verlauf neues Knochenmaterial gebildet und somit beispielsweise ein aufgrund von Parodontitis vorgeschädigter Kieferknochen wieder aufgebaut werden kann.

Das vom Körper vollständig resorbierbare Formteil ist dabei dauerhaft formstabil ausgebildet und so bemessen, dass erst nach ausreichender Ossifikation bzw. Osteogenese, d.h. wenn keine stützende Struktur mehr benötigt wird, der Resorptionsprozess abgeschlossen wird. Bevorzugt lässt sich das erfindungsgemäße Formteil als Massenprodukt in unterschiedlichen Größen herstellen. Dabei ist das Formteil derart ausgeformt, das es ohne Veränderungen, abgesehen von kleineren Korrekturen direkt auf oder an dem Knochen verwendet werden kann. Das Formteil wird hierzu in verschiedenen Größen und an verschiedene Verwendungspositionen angepasst zur Verfügung gestellt. vom menschlichen oder tierischen Körper vollständig resorbierbar ausgebildet das Formteil ist

Die ebenfalls im Zusammenhang mit der vorliegenden Erfindung verwendbare Hyaluronsäure (bzw. Hyaluronsäure-Derivate) wirkt sich günstig auf die Behandlung pathologischer Veränderungen des Parodontiums aus und zeigt positive Effekte auf Fibroblasten, Knochenregeneration und Wundheilung. Im Zusammenhang mit der vorliegenden Erfindung kann Hyaluronsäure (bzw. deren Derivate) direkt in der erfindungsgemäßen Zusammensetzung beigegeben oder beigemischt werden. Alternativ kann nach Vorbereitung des Formteils und während des Einsetzens bzw. des Auflegens auf einer Knochenunterlage die Beigabe oder Spülung des Einsatzortes mit einem Hyaluronsäurepräparat erfolgen. Hyaluronsäure hat dabei verschiedene Funktionen.

Das grundsätzliche Wirkungsprinzip von Hyaluronsäure im Zusammenhang mit der vorliegenden Erfindung sieht vor, dass es in wässriger Umgebung in Folge einer spontanen Aggregation der Hyaluronsäureketten zur Entstehung dreidimensionaler Maschennetzwerke kommt. In dieses können zelluläre und fibröse Komponenten eingebettet werden. Hierdurch wird die Ausbildung einer Knochenstruktur begünstigt und gefördert. Gleichzeitig besitzt Hyaluronsäure eine regulierende Funktion bei der Organisation der extrazellulären Matrix und ihrer Bestandteile. Das gebildete Hyaluronsäurenetzwerk bildet dabei eine Voraussetzung für den Stoffaustausch und dient gleichzeitig als Barriere gegen das Eindringen fremder Substanzen. Durch die Bildung der Netzwerke und deren Kondensierung können Zellen vor Abbauprozessen und Hydroxylradikalen geschützt werden. Die somit vorhandenen Hüllen aus Hyaluronsäure dienen verschiedenen Zelltypen als Schutz vor äußeren, bspw. viralen oder bakteriellen Einflüssen und begünstigen somit auch die Überlebenswahrscheinlichkeit der Osteoblasten.

Negativ geladene Hyaluronsäure hat zudem die Fähigkeit, enorme Mengen an Wasser und verschiedenen Plasmaproteinen über Wasserstoffbrückenbindung und die polaren Enden zu binden und fungiert somit als eine Art "osmotischer Puffer" der extrazellulären Matrix. Hyaluronsäure erweist sich auch bei der Bekämpfung chronischer Entzündungsherde und als vorteilhaft und weist ein antiinflammatorisches Potential auf. Hyaluronsäure beeinflusst auch zelluläre Wachstumsfaktoren und hat somit einen positiven Einfluss auf zelluläre Wachstumsprozesse und unterstützt somit die Geweberegenerierung. Diese zahlreichen Vorteile werden im Zusammenhang mit der vorliegenden Erfindung bzw. der Zusammensetzung genutzt. Überraschend hat sich gezeigt, dass die Regeneration des Knochens bzw. Knochenmaterials wesentlich verbessert werden kann.

Überraschend hat sich gezeigt, dass das Formteil der vorliegenden Erfindung eine gegenüber dem Stand der Technik klar überlegene Form der Ossifikation bzw. Osteogenese ermöglicht.

Dass im Formteil bzw. der Zusammensetzung vorgesehene Granulat ist aus einem Grundmaterial ausge-wählt, das ausgewählt ist aus der Gruppe bestehend aus Aragonit, Muschelschale, allogenem Knochenmaterial, autogenem Knochenmaterial, xenogenem Knochenmaterial, FDBA (freeze-dried bone allocrafts), DFBDA (decalzified freeze-dried bone allocrafts), Algen oder Algenextrakt, Keramik, Calciumphosphat, insbesondere Tri- oder Tetracalciumphosphat, α- oder β-Tricalciumphosphat, Hydroxyl-Apatit, Calciumphosphat-Keramik, Bioglas, Knochenersatzmaterial auf Aragonitbasis (z.B. BioCoral ^{®}) oder Mischungen daraus.

Insbesondere ist es denkbar, das Granulat aus Spenderknochen herzustellen. Granulat, das aus Knochen aus Knochenbanken hergestellt ist ist gleichfalls von der Erfindung umfasst.

Die Erfindung sieht auch die Verwendung von FDBA (freeze dried bone allacrafts) oder von DFDBA (decalcified freeze dried bone allocrafts) als vorteilhaft an. Durch die Ausbildung des Granulates aus einem aus einem genetisch differenten Individuum derselben Spezies entnommenen Material, kann das Knochenwachstum optimal verlaufen. Die Wahrscheinlichkeit entzündlicher Reaktionen wird vorteilhaft reduziert. Auch die Verwendung von xenogenem Materialien zur Herstellung des Granulates erweist sich als günstig.

Zur Herstellung von Granulaten, die für den Menschen geeignet sind, eignen sich insbesondere Knochen von Rind, Schwein und Pferd. Es ist auch möglich und von der Erfindung umfasst, das Granulat aus Algen, insbesondere Algenextrakten, Korallen oder Muscheln zur Verfügung zu stellen. Als besonders geeignet für die Herstellung des Granulates erweisen sich die Schalen von Muscheln, da sie aus einem Calcium-Protein-Gemisch, genauer aus Aragonit bestehen und daher besonders gut vom Körper resorbiert werden können.

Daneben ist es auch möglich, das Granulat aus autogenem, d.h. vom Patienten selbst zur Verfügung gestelltem Material herzustellen. Dem Patienten wird hierzu zunächst Knochenmaterial entnommen, dieses zu Granulat verarbeitet und für die Verwendung im erfindungsgemäßen Formteil, das dem Patienten im Rahmen einer weiteren Behandlung eingesetzt bzw. implantiert wird, vorbereitet. Hierbei ist die Wahrscheinlichkeit des Auftretens von entzündlichen Reaktionen im Körper des Patienten am geringsten.

Weiterhin ist es möglich, alloplastische Materialien wie Calciumphosphate, Keramiken oder Biogläser für die Herstellung des Granulates zu verwenden.

Bevorzugt besteht das Grundmaterial des Granulates: Aragonit in Kombination mit zwischen 0 und 50 %, insbesondere zwischen 15 und 35 %, bevorzugt 25 % Knochenmaterial, insbesondere allogenem oder autogenem Knochenmaterial. Die Verwendung von xenogenem Knochenmaterial oder einem oder mehreren der sonstigen oben ausgeführten Materialien ist gleichfalls möglich und von der Erfindung umfasst. Auch umfasst sind Kombinationen oder Mischungen verschiedener Materialien und deren Verwendung in Kombination mit Aragonit.

Vorteilhaft ist, wenn das Grundmaterial des Granulates nur aus Knochenmaterial, insbesondere allogenem, autogenem und/oder xenogenem Knochenmaterial gebildet ist.

Das Granulat weist eine Korngröße von zwischen 1 und 3 mm, insbesondere von zwischen 1,1 und 2 mm, bevorzugt von 1,5 mm auf. Diese Korngrößen bzw. Korngrößenbereiche erweisen sich unter Resorptionsgesichtspunkten als optimal. Durch auf den jeweiligen Patienten bzw. Einsatzzweck abgestimmte Auswahl der Korngröße kann die Resorptionsdauer und -geschwindigkeit definiert und der Behandlungserfolg somit weiter verbessert werden. Neben der Korngröße ist auch die Porosität des Granulatmaterials ein zu beachtendes Kriterium. Eine hohen Anzahl von Poren bzw. Porenkörpern im Granulat bzw. auf der Granulatoberfläche kann die für das Aufwachsen von Gefäßen bzw. Osteoblasten zur Verfügung stehende Fläche wesentlich vergrößern und das Osteoblastenwachstum dadurch verbessert werden. Die Porosität des Granulatmaterials ergibt sich zum Einen aus dem Material selbst bzw. kann zum Anderen durch geeignete Vorbehandlung des Granulats oder Granulatausgangsmaterials, bzw. durch eine Säurebehandlung oder dergleichen, in einem definierten Bereich eingestellt werden.

Als vorteilhaft erweist sich, wenn zwischen dem Formteil und der Knochenunterlage ein Dichtungsmaterial vorgesehen ist, um das Auswachsen von Gefäßen bzw. das Eindringen von das Knochenwachstum schädigenden Substanzen oder Mikroorganismen in das Formteil an unerwünschten Stellen zu unterbinden. Das Dichtungsmaterial wird hierbei insbesondere aus Collagen, bevorzugt Collagen Typ 1 oder Typ 3 oder eine Mischung aus Collagen Typ 1 und Collagen Typ 3 und/oder Hyaluronsäure bzw. Hyaluronsäure-Derivat gebildet.

In einer als vorteilhaft angesehenen Weiterbildung der Erfindung ist vorgesehen, dass die das Formteil bildende Zusammensetzung wenigstens eine weitere Substanz beinhaltet. Diese ist bevorzugt ausgewählt aus der Gruppe bestehend aus Statin, Vitaminen, Spurenelementen, Antibiotika oder Mischungen daraus. Während Vitamine und Spurenelemente der Versorgung der neugebildeten Zellen dienen, begünstigen Statine bzw. Statinpräparate die Immunmodulation und verringern so die Entzündungsneigung. Antibiotika dienen der Bekämpfung bzw. Vermeidung bakterieller Infektionen an oder in der Knochenunterlage. Die Erfindung bleibt nicht auf die vorgenannten Substanzen beschränkt, sondern schließt alle dem Fachmann geläufigen und im Zusammenhang mit der vorliegenden Erfindung verwendbaren Substanzen und Substanzgemische ein.

In diesem Zusammenhang erweist es sich als vorteilhaft, wenn die wenigstens eine weitere Substanz einen Anteil von zwischen 0,1 - 3 %, insbesondere von zwischen 0,2 - 1,5 %, bevorzugt 0,25 % an der Zusammensetzung aufweist.

Erfindungsgemäß ist das Formteil in der grundlegenden Ausgestaltung aus dem wie zuvor definierten strukturgebenden Material und dem ebenfalls zu vordefinierten Granulat gebildet. Im Formteil ist ein Verhältnis von strukturgebendem Material zu Granulat von zwischen 1:1 und 1:4 vorgesehen. Als besonders günstig angesehen wird ein Formteil, das aus 1 Teil des strukturgebenden Materials und 2 Teilen Granulat gebildet ist. Abhängig vom Einsatzzweck und von den Gegebenheiten an der Stelle, an der neuer Knochen gebildet werden soll, können die vorgenannten Verhältnisse selbstverständlich auch abweichend eingestellt werden. Das für die Herstellung der modellierbaren Masse aus der Zusammensetzung benötigte Wasser, bevorzugt destilliertes und sterilisiertes Wasser, bleibt unberücksichtigt.

Überraschend hat sich gezeigt, dass ein Verhältnis von strukturgebendem Material zu Granulat von 1:2 die besten Erfolge gezeigt hat, wenn es darum ging ein ausreichend stabiles Knochenmaterial aufwachsen zu lassen. Neben der durch das so ausgeführte Formteil zur Verfügung gestellten Struktur für das Knochenwachstum bzw. die Ossifikation gewährleistet ein entsprechendes Verhältnis von strukturgebenden Material und Granulat auch eine zeitlich definierte Resorption des Materials.

Das biokompatible Formteil ist block- oder quaderförmig ausgebildet. Daneben besteht es verständlich auch die Möglichkeit, das Formteil in seiner Form an eine Ausnehmung im Knochen, insbesondere Kieferknochen oder Kieferknochenabschnitt angepasst auszubilden. Wird ein standardisiertes Formteil zur Verfügung gestellt, so kann dieses vor dem Auflegen auf dem Knochen in seiner Form, das heißt insbesondere in seiner Höhe, Breite und Länge angepasst werden. Somit wird gewährleistet, dass ein an die Gegebenheiten im Patienten angepasstes Formteil zur Verfügung gestellt wird. Weiterer Vorteil ist, dass ein eine einheitliche Form aufweisendes Formteil standardisiert und damit kostengünstiger hergestellt werden.

Das block- oder quaderförmige oder in seiner Form an eine Ausnehmung im weist Kieferknochen oder Kieferknochenabschnitt angepasste Formteil Kanten mit einer Kantenlänge von jeweils zwischen 1 und 5 cm aufweist. Als günstig wird angesehen, wenn die Kantenlänge zwischen 1,5 und 3 cm beträgt. Ein solches standardisiertes Formteil weist bevorzugt eine Breite von maximal 1,5 cm eine Länge von maximal 3 cm und eine Höhe von maximal 1,5 cm auf. Die entsprechenden Kantenlängen und die daraus resultierende Größe und das Volumen des Formteils gewährleisten, dass ein vollständiges Durchwachsens mit Gefäßen und eine ausreichend stabile Osteogenese erfolgen kann. Gleichzeitig wird eine vollständige Resorption des Formteils im Verlauf des Heilungsprozesses erreicht.

In einer Weiterbildung des erfindungsgemäßen biokompatiblen Formteils wird es als günstig angesehen, wenn dieses Formteil wenigstens eine Bohrung aufweist. Diese Bohrung dient dem Durchgriff eines Befestigungsmittels, insbesondere einer Schraube, die für die Befestigung des Formteils auf dem Knochen verwendet wird. Die Bohrung, bzw. die Bohrungen werden dabei bereits bei der Herstellung, das heißt Modellierung des Formteils in dieses eingebracht. Alternativ besteht selbstverständlich auch die Möglichkeit, dass diese Bohrungen mit geeigneten Werkzeugen nachträglich in dem Formteil eingefügt werden. Auch kann eine zuvor definierte bzw. individuell angepasste Anordnung im Formteil erfolgen, die auf die späteren Anordnungspunkte des Formteils am Knochen abgestimmt ist.

Von gleicher erfinderischer Bedeutung ist eine Zusammensetzung zur Herstellung des biokompatiblen Formteils. Diese umfasst oder besteht aus einem strukturgebenden Material und einem Granulat. Strukturgebendes Material und Granulat werden Ihnen definierten Verhältnissen gemischt, um hieraus dann, nach Zugabe von Wasser, ein Formteil, insbesondere gemäß der vorliegenden Erfindung zu modellieren.

Das strukturgebende Material ist bevorzugt ausgewählt aus der Gruppe bestehend aus Alabastergips, Hartgips, Superhartgips, oder Mischungen daraus. Alle vorgenannten Materialien können vollständig resorbiert werden und sind aus der Verwendung im kieferorthopädischen und implantationsmedizinischen Bereich bekannt und hierfür geeignet. Bevorzugt wird Alabastergips verwendet, der neben einer entsprechenden Resorbierbarkeit eine ausreichende Stabilität des Formteils bzw. Vollkörpers gewährleistet. Der Begriff Alabastergips ist ein aus der Zahnkunde hinlänglich bekannter Begriff. Bezüglich seiner Härte liegt Alabastergips zwischen dem ebenfalls im zahntechnischen Bereich verwendeten Abdruckgips sowie einem Hartgips. Dieser Gips kann nach Herstellung des Formteils weiter bearbeitet werden, und lässt somit eine Anpassung des Vollkörpers an die Gegebenheiten im Kiefer mit einfachen Werkzeugen zu.

Als vorteilhaft wird angesehen, wenn dass Granulat aus einem Grundmaterial ausgewählt ist aus der Gruppe bestehend aus Aragonit, Muschelschale, allogenem Knochenmaterial, autogenem Knochenmaterial, xenogenem Knochenmaterial, FDBA (freeze-dried bone allocrafts), DFBDA (decalzified freeze-dried bone allocrafts), Algen oder Algenextrakt, Keramik, Calciumphosphat, insbesondere Tri- oder Tetracalciumphosphat, α- oder β-Tricalciumphosphat, Hydroxyl-Apatit, Calciumphosphat-Keramik, Bioglas, Knochenersatzmaterial auf Aragonitbasis (z.B. BioCoral ^{®}) oder Mischungen daraus.

Insbesondere ist es denkbar, das Granulat aus Spenderknochen herzustellen. Granulat das aus Knochen aus Knochenbanken hergestellt ist ebenfalls von der Erfindung umfasst.

Die Erfindung sieht auch die Verwendung von FDBA (freeze dried bone allocrafts) oder von DFDBA (decalcified freeze dried bone allocrafts) als vorteilhaft an. Durch die Ausbildung des Granulates aus einem aus einem genetisch differenten Individuum derselben Spezies entnommenen Material kann das Knochenwachstum optimal verlaufen. Die Wahrscheinlichkeit entzündlicher Reaktionen wird vorteilhaft reduziert. Auch die Verwendung von xenogenen Materialien zur Herstellung des Granulates erweist sich als vorteilhaft.

Zur Herstellung Granulaten, die für den Menschen geeignet sind, eignen sich insbesondere Knochen von Rind, Schwein und Pferd. Es ist auch möglich und von der Erfindung umfasst, das Granulat aus Algen, insbesondere Algenextrakten, Korallen oder Muscheln herzustellen. Als besonders geeignet für die Herstellung des Granulates erweisen sich die Schalen von Muscheln, da sie aus einem Calcium-Protein-Gemisch, genauer aus Aragonit bestehen und daher besonders gut vom Körper resorbiert werden können.

Daneben ist es auch möglich, das Granulat aus autogenem, d.h. vom Patienten selbst zur Verfügung gestelltem Material herzustellen. Dem Patienten wird hierzu zunächst Knochenmaterial entnommen, dieses zu Granulat verarbeitet und für die Verwendung im erfindungsgemäßen Formteil, das dem Patienten im Rahmen einer weiteren Behandlung eingesetzt bzw. implantiert wird, vorzubereitet. Hierbei ist die Wahrscheinlichkeit des Auftretens von entzündlichen Reaktionen im Körper des Patienten am geringsten.

Weiterhin ist es möglich, alloplastische Materialien wie Calciumphosphate, Keramiken oder Biogläser für die Herstellung des Granulates zu verwenden.

Das Granulat weist eine Korngröße von zwischen 1 und 3 mm, insbesondere von zwischen 1,1 und 2 mm, bevorzugt von 1,5 mm auf. Diese Korngrößen bzw. Korngrößenbereiche erweisen sich unter Resorptionsgesichtspunkten als optimal. Durch auf den jeweiligen Patienten bzw. Einsatzzweck abgestimmte Auswahl der Korngröße kann die Resorptionsdauer und -geschwindigkeit definiert und der Behandlungserfolg somit weiter verbessert werden. Neben der Korngröße ist auch die Porosität des Granulatmaterials ein zu beachtendes Kriterium. Eine hohen Anzahl von Poren bzw. Porenkörpern im Granulat bzw. auf der Granulatoberfläche kann die für das Aufwachsen von Gefäßen bzw. Osteoblasten zur Verfügung stehende Fläche wesentlich vergrößern und deren Wachstum dadurch verbessert werden. Die Porosität des Granulatmaterials ergibt sich zum Einen aus dem Material selbst bzw. kann zum Anderen durch geeignete Vorbehandlung des Granulats oder Granulatausgangsmaterials, bzw. durch eine Säurebehandlung oder dergleichen, in einem definierten Bereich eingestellt werden.

In einer als vorteilhaft angesehenen Weiterbildung der Erfindung ist vorgesehen, dass die Zusammensetzung wenigstens eine weitere Substanz beinhaltet. Diese ist bevorzugt ausgewählt aus der Gruppe bestehend aus Statin, Vitaminen, Spurenelementen, Antibiotika oder Mischungen daraus. Während Vitamine und Spurenelemente der Versorgung der neugebildeten Zellen dienen, begünstigen Statine bzw. Statinpräparate die Immunmodulation und verringern die Entzündungsneigung. Antibiotika dienen der Bekämpfung bzw. Vermeidung bakterieller Infektionen an oder in der Knochenunterlage. Die Erfindung bleibt nicht auf die vorgenannten Substanzen beschränkt, sondern schließt alle dem Fachmann geläufigen und im Zusammenhang mit der vorliegenden Erfindung verwendbaren Substanzen und Substanzgemische ein.

In diesem Zusammenhang erweist es sich als vorteilhaft, wenn die wenigstens eine weitere Substanz einen Anteil von zwischen 0,1 - 3 %, insbesondere von zwischen 0,2 - 1,5 %, bevorzugt 0,25 % an der Zusammensetzung aufweist.

Bevorzugt weist das Granulat oder das Grundmaterial eine umhüllende Schicht aus wenigstens einem Collagen, Hyaluronsäure und/oder Hyaluronsäure-Derivat oder Mischungen daraus auf.

Aus der Beschichtung des Granulats oder Grundmaterials ergeben sich weitere Vorteile. So wird Blut des Patienten aufgesaugt, sodass Körperzellen an jedem Ort innerhalb des und auf dem Formteil verfügbar sind. Das Wachstum der in das Formteil einwachsenden Gefäße wird ebenfalls gefördert, da diese Gefäße für die dauerhafte Nährstoffversorgung der Osteoblasten entscheidend sind. Nur bei ausreichender Nährstoffversorgung kann neues Knochenmaterial gebildet werden kann.

Die ebenfalls im Zusammenhang mit der vorliegenden Erfindung verwendbare Hyaluronsäure (bzw. Hyaluronsäure-Derivate) wirkt sich günstig auf die Behandlung pathologischer Veränderungen des Parodontiums aus und zeigt positive Effekte auf Fibroblasten, Knochenregeneration und Wundheilung. Im Zusammenhang mit der vorliegenden Erfindung wird Hyaluronsäure (bzw. deren Derivate) direkt auf das Granulat aufgebracht. Die Hyaluronsäure hat dabei verschiedene Funktionen. Das grundsätzliche Wirkungsprinzip der Hyaluronsäure im Zusammenhang mit der vorliegenden Erfindung sieht vor, dass es in wässriger Umgebung in Folge einer spontanen Aggregation der Hyaluronsäureketten zur Entstehung dreidimensionaler Maschennetzwerke kommt. In dieses können zelluläre und fibröse Komponenten eingebettet werden. Hierdurch wird die Ausbildung einer Knochenstruktur begünstigt und gefördert. Gleichzeitig besitzt Hyaluronsäure eine regulierende Funktion bei der Organisation der extrazellulären Matrix und ihrer Bestandteile. Das gebildete Hyaluronsäurenetzwerk bildet dabei eine Voraussetzung für den Stoffaustausch und dient gleichzeitig als Barriere gegen das Eindringen fremder Substanzen. Durch die Bildung der Netzwerke und deren Kondensierung können Zellen vor Abbauprozessen und Hydroxylradikalen geschützt werden. Die somit vorhandenen Hüllen aus Hyaluronsäure dienen verschiedenen Zelltypen als Schutz vor äußeren, bspw. viralen oder bakteriellen Einflüssen und begünstigen somit auch die Überlebenswahrscheinlichkeit der Osteoblasten.

Negativ geladene Hyaluronsäure hat zudem die Fähigkeit, enorme Mengen an Wasser und verschiedenen Plasmaproteinen über Wasserstoffbrückenbindung und die polaren Enden zu binden und fungiert somit als eine Art "osmotischer Puffer" der extrazellulären Matrix. Hyaluronsäure erweist sich auch bei der Bekämpfung chronischer Entzündungsherde und als vorteilhaft und weist ein antiinflammatorisches Potential auf. Hyaluronsäure beeinflusst auch zelluläre Wachstumsfaktoren und hat somit einen positiven Einfluss auf zelluläre Wachstumsprozesse und unterstützt somit die Geweberegenerierung. Diese zahlreichen Vorteile werden im Zusammenhang mit der vorliegenden Erfindung genutzt. Überraschend hat sich gezeigt, dass die Regeneration des Knochens bzw. Knochenmaterials wesentlich verbessert werden kann. Es wird damit eine gegenüber dem Stand der Technik klar überlegene Form der Ossifikation bzw. Osteogenese bewirkt, was unter anderem aus der erfindungsgemäßen Zusammensetzung und der enthaltenen bzw. freigegebenen Hyaluronsäure in Kombination mit den übrigen Bestandteilen realisiert.

Erfindungsgemäß ist die Zusammensetzung aus dem wie zuvor definierten strukturgebenden Material und dem ebenfalls zuvor definierten Granulat gebildet. Als günstig wird in diesem Zusammenhang angesehen, wenn in der Zusammensetzung ein Verhältnis von strukturgebendem Material zu Granulat von zwischen 1:1 und 1:4 vorgesehen ist. Als besonders günstig wird eine Zusammensetzung angesehen, die zu einem Teil aus strukturgebendem Material und zu zwei Teilen aus Granulat gebildet ist. Abhängig vom Einsatzzweck können die vorgenannten Verhältnisse selbstverständlich auch umgekehrt oder geändert vorliegen. Überraschend hat sich jedoch gezeigt, dass ein Verhältnis von strukturgebendem Material zu Granulat in der Zusammensetzung von 1:2 die besten Erfolge gezeigt hat. Über das definierte Verhältnis von strukturgebendem Material zu Granulat kann die Resorption des Materials gesteuert werden.

Insgesamt sind jedoch vorteilhafterweise sämtliche Komponenten der Zusammensetzung durch den menschlichen oder tierischen Körper resorbierbar.

Bevorzugt besteht das Grundmaterial des in der Zusammensetzung verwendbaren Granulates aus Aragonit in Kombination mit zwischen 0 und 50%, insbesondere zwischen 15 und 35 %, bevorzugt 25 % Knochenmaterial, insbesondere allogenem oder autogenem Knochenmaterial. Die Verwendung von xenogenem Knochenmaterial oder einem oder mehreren der sonstigen oben ausgeführten Materialien ist gleichfalls möglich und von der Erfindung umfasst. Auch umfasst sind Kombinationen verschiedener Materialien und deren Verwendung in Kombination mit Aragonit.

Die Erfindung umfasst auch ein Verfahren zur Herstellung eines biokompatiblen Formteils. Das Verfahren umfasst dabei die folgenden Schritte:
(i) Herstellen einer formbaren Modelliermasse durch Anmischen der wie zuvor definierten Zusammensetzung. Hierfür wird Wasser, bevorzugt destilliertes und steriles Wasser verwendet.
(ii) Modellieren des Formteils als block- oder quaderförmiges Formteil und
(iii)Trocknen des Formteils.

Das Formteil wird hierbei bevorzugt als standardisiertes Teil hergestellt und eignet sich so besonders zur Serienproduktion. Das im Verfahren hergestellte Formteil kann bei Verwendung an die jeweiligen Gegebenheiten im Kieferknochen des Patienten angepasst werden.

Die Trocknung erfolgt bei Raumtemperatur oder in einem speziellen Trockenofen bei erhöhter Temperatur. In einer Ausführungsform der Erfindung ist ein Brennen des Formteils vorgesehen.

Die Schwindung des Formteils im Verlauf des Trocknens wird bei der Herstellung/Modellierung als Übermaßaufschlag berücksichtigt

Optional oder alternativ kann das Verfahren auch die folgenden Schritte umfassen:
(ia) Ermitteln einer Ausformung einer Knochenstruktur, insbesondere der Form des Kieferknochens oder Kieferknochenabschnittes in oder an der das Formteil an- oder eingebracht werden soll, und
(iia) Modellieren des Formteils anhand der ermittelten Ausformung

Die der Form des Kieferknochens oder Kieferknochenabschnittes in oder an der das Formteil an- oder eingebracht werden soll wird dabei beispielsweise aus einer Röntgenaufnahme abgeleitet.

Das Formteil wird vor dem Einsetzen bereits an die jeweiligen Fehlstellen angepasst bzw. auf diese abgestimmt hergestellt. Es wird so ein nachträgliches Bearbeiten des fertiggestellten Formteils umgangen und ein passgenaues Formteil zur Verfügung gestellt.

Um die Anpassung des Formteils weiter zu verbessern, kann zuvor das optionale Erstellen eines Negativmodells der Knochenstruktur durchgeführt werden.

Um das Aufwachsen von Keimen zu verhindern und somit Entzündung zu vermeiden, bzw. um die Keimzahl zu reduzieren, wird es als vorteilhaft angesehen wenn das Verfahren weiter den Schritt (iv) Sterilisieren des Formteils, insbesondere durch Bestrahlung mit Gammastrahlung umfasst. Selbstverständlich ist auch eine Hitzesterlisierung möglich.

Die Herstellung des Formteils wird wesentlich dadurch vereinfacht, dass für das Modellieren eine Form, bevorzugt eine Silikonform verwendet wird. Diese Form kann in verschiedenen Größen hergestellt und zur Verfügung gestellt werden. Bei der Herstellung wird die Form mit der aus der erfindungsgemäßen Zusammensetzung unter Verwendung von Wasser hergestellten Modelliermaße verfüllt. Nach Entnahme aus der Form werden die Formteile getrocknet, gegebenenfalls bearbeitet (Glättung, Bohrung, Fräsung, Brechung der Kanten etc.), anschließend sterilisiert und dann verpackt.

Um die Befestigung des Formteils am Kiefer beziehungsweise Kieferknochen zu ermöglichen, wird es als vorteilhaft angesehen vor oder nach dem Trockenen wenigstens eine durchgehende Bohrung in das Formteil einzubringen. Diese Bohrung(en) dient(en) dann dem Durchgriff von Schrauben, über die das Formteil mit dem Kiefer verbunden wird. Nach der Ausbildung des Knochens und der Resorption des Formteils können diese Schrauben dann aus dem neugebildeten Knochen entnommen werden.

Die Erfindung stellt auch eine Verwendung eines wie zuvor definierten Formteils vor. Insbesondere geeignet ist das Formteil für die Verwendung in der plastischen Medizin oder der Zahnmedizin. Bevorzugt erfolgt die erfindungsgemäße Verwendung zur Unterstützung einer Knochenneubildung, insbesondere im Kieferknochen, wobei das Formteil den sich neu bildenden Knochen stabilisiert und anschließend beziehungsweise im Verlauf der Knochenneubildung vollständig resorbiert und dabei von neugebildetem Knochen ersetzt wird.

Als vorteilhaft wird in diesem Zusammenhang die Verwendung des Formteils zur Knochenaugmentation einer vorhandenen Knochenstruktur angesehen. Insbesondere eine Knochenaugmentation von bis zu ca. 1,5 cm ist dabei möglich.

Bei Verwendung des Formteils wird es als vorteilhaft angesehen, wenn vor dem Einsatz eine Längen-, Breiten- und/oder Höhenanpassung des Formteils durchgeführt wird.

Die Erfindung umfasst auch ein Kit das mehrere Formteile wie zuvor beschrieben umfasst. Die Formteile weisen hierbei bevorzugt gleiche oder unterschiedliche Größen und/oder Ausformungen auf. Bei Verwendung kann somit das am besten geeignetste Formteil aus dem Kit ausgewählt und für den Aufbau des Kieferknochens eingesetzt werden. Dabei kann das von der Größer und Ausformung her am besten passende Formteil ausgewählt und dann eine Längen-, Breiten- und/oder Höhenanpassung der Formteil vor Verwendung durchgeführt werden. Die Überarbeitung des vorgefertigten Formteils erfolgt dann mit herkömmlichen Werkzeugen. Die Größe kann durch einfaches Abschleifen überschüssigen Materials angepasst werden. Das erfindungsgemäße Kit ermöglicht es dem Anwender das am besten geeignetste Formteil auszuwählen, so dass der Anpassungsbedarf nur sehr gering ist.

### Beispiel:

Die durch das erfindungsgemäße Formteil induzierte, vorteilhafte Ossifikation bzw. Osteogenese konnte im Tierversuch nachgewiesen werden. Im Tierexperiment wurde drei Schweinen blockförmige Formteile, nachfolgend bezeichnet als Formteil 1 bzw. Formteil 2 an der Schädelkalotte implantiert.

Die Formteile wiesen dabei folgende Merkmale auf:
Formteil A:
Gewicht: ca. 15g
Zusammensetzung:
2/3 Gips als strukturgebendes Material
1/3 Granulat gemäß der Erfindung

Abmessungen des Formteils:

| | |
|---|---|
| Länge: | 2 cm |
| Breite: | 1 cm |
| Höhe: | 1,5 cm |

Formteil B:
Gewicht: ca. 15g
Zusammensetzung:
2/3 Carboxylat-Zement als strukturgebendes Material
1/3 Granulat gemäß der Erfindung

Abmessungen des Formteils:

| | |
|---|---|
| Länge: | 2 cm |
| Breite: | 1 cm |
| Höhe: | 1,5 cm |

Die oben genannten Formteile wurden an den Schädelkalotten bei drei Schweinen implantiert. Formteil A auf der linken Seite, Formteil B auf der rechten Seite des jeweiligen Tiers. Zur Befestigung der Formteile an der Kalotte wurden in diese durchgehende Bohrungen eingebracht. Diese Bohrungen dienten dem Durchgriff von Schrauben, über die die Formteile mit der Kalotte verschraubt wurden.

Nach 8 Wochen wurden die Tiere geopfert und die implantierten Stellen histologisch untersucht.

Ergebnis:
An den Stellen, an denen das Formteil A implantiert worden war hatte der Abbau des Materials und dessen Resorption begonnen. Gleichzeitig setzte dort nachweisbare Knochenregeneration bzw. Knochenneubildung ein. Es zeigten sich eine gute Einheilung und ein vertikaler Höhengewinn. Die Schrauben waren im gebildeten Knochen von Knochenmaterial umgeben.

An den Stellen, an denen das Formteil B implantiert worden war kam es während des Versuchszeitraums zu verringertem bis ausbleibendem Abbau des Materials. Auch konnte dort keine Knochenregeneration bzw. Knochenneubildung nachgewiesen werden. Es traten partiell Wundheilungsstörungen auf.

Weitere Vorteile und zweckmäßige Ausführungen sind der Figurenbeschreibung und der Zeichnung zu entnehmen. Es zeigt:
Fig. 1 eine schematische Darstellung einer bevorzugten Ausführungsform einen Formteils, und
Fig. 2 die computertomographische Auswertung des oben beschriebenen Bespielversuchs.

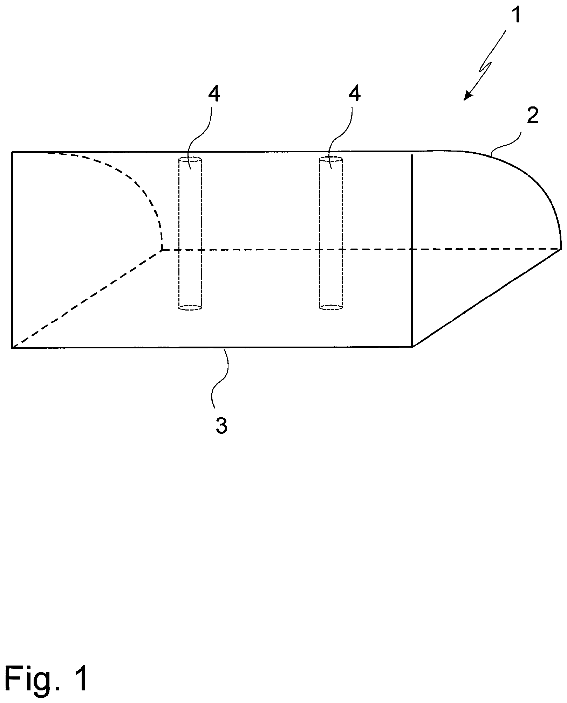

In Fig.1 ist das Formteil 1 perspektivisch dargestellt. Das Formteil 1 weist oberseits eine Abrundung 2 auf, die der Form des Kieferknochen nachempfunden ist. Die weiteren Abschlussflächen 3 des Formteils 1 nehmen zueinander rechte Winkel ein. Es ergibt sich somit ein block- oder quaderförmiges Formteil 1. Dieses hier als Standardelement dargestellte Formteil 1 kann vor der Verwendung auf die jeweiligen Gegebenheiten im Kiefer des Patienten abgestimmt werden. Hierdurch können die Abschlussflächen 3 entsprechend beschnitten oder abgeschliffen werden. Hierbei kann dann auch eine Anpassung der Größe des Formteils 1 durchgeführt werden. Das Formteil 1 in der in Figur 1 dargestellten Ausführungsform weist eine Länge von 3 cm, eine Höhe von 1,5 cm und eine Breite von ebenfalls 1,5 cm auf. Das Formteil 1 ist aus einer Zusammensetzung, die aus Alabastergips und einem aus einem Knochenmaterial gebildeten Granulat hergestellt ist gebildet. Die Zusammensetzung wurde mit destilliert Wasser angemischt, bis sich eine Masse mit einer modellierbaren Konsistenz ergab. Anschließend erfolgte ein Verfüllen einer Silikonform mit der Modelliermasse. Die Silikonform weist dabei die Form des fertigen Formteils 1 auf. Nach Entnahme des Formteils 1 aus der Form wurde dieses getrocknet. Die sich bei Trocknung ergebende Schrumpfung des Materials wurde bei der Dimensionierung der Silikonform berücksichtigt. Nach Sterilisieren und Verpacken wurde das Formteil 1 für die Verwendung zur Verfügung gestellt. Vor dem Einsetzen bzw. Aufsetzen auf dem Kieferknochen erfolgt eine abschließende Bearbeitung des Formteils 1. Zusätzlich sind im Formteil zwei Bohrungen 4 vorgesehen. Über diese Bohrungen 4 kann mittels Knochenschrauben eine Befestigung des Formteils 1 auf dem Kieferknochen (nicht dargestellt) durchgeführt werden. Nach Resorption des Formteils 1 und Abschluss der Knochenneubildung können die Schrauben wieder aus dem Kiefer entnommen werden. Die verbleibenden Öffnungen werden beispielsweise mit einem ebenfalls die Osteogenese fördernden Material verfüllt und nachträglich ossifiziert.

Um zu verhindern, dass Zellen der Knochenhaut oder Zellen des Zahnfleisches in das Formteil 1 unerwünscht eindringen, kann das Formteil 1 gegenüber den umliegenden Zähnen mithilfe von Collagen abgedichtet werden.

Um das Formteil 1 am Kieferknochen anzubringen, wird zunächst der obere Zahnfleischlappen aufgeklappt. Gegebenenfalls wird die Oberfläche des Kieferknochens aufgeraut, um das Wachstum des Knochens zu fördern. Daraufhin wird das Formteil 1 an der entsprechenden Stelle aufgebracht und am Kieferknochen mit Stiften oder Schrauben fixiert. Daraufhin wird der Zahnfleischlappen über das Formteil 1 geklappt und an der Außenseite des Formteils 1 fixiert. An der Außenseite des Formteils 1 wächst dann die Knochenhaut entlang, so dass nach einiger Zeit die ursprüngliche Kiefersituation mit vollständigem Kieferknochen, Knochenhaut und Zahnfleisch wiederhergestellt ist. Eine zweite Operation zur Entfernung des Formteils 1 nach erfolgter Knochenneubildung ist nicht erforderlich, da das Formteil 1 vom Körper vollständig abgebaut wird.

Blutgefäße und Knochenzellen wachsen in das Formteil 1 ein und durchsetzen dieses nach und nach vollständig. Der Kieferknochen wird dabei sukzessive remodelliert. Insgesamt stellt das eingesetzte Formteil 1 eine Struktur zur Verfügung, die als Basis oder Gerüst für die Gefäßneubildung dient. Diese Basis führt letztendlich zu einer Knochenneubildung, wenn Osteoblasten eine ausreichende Nährstoffversorgung vorfinden, um neues Knochenmaterial zu bilden. Das Formteil 1 wird vollständig vom Körper resorbiert. Die gesamte Einsatzstelle des Formteils 1 kann mit einer Hyaluronsäurelösung gespült werden, die in der ersten Einwachsphase das Gefäßwachstum begünstigt.

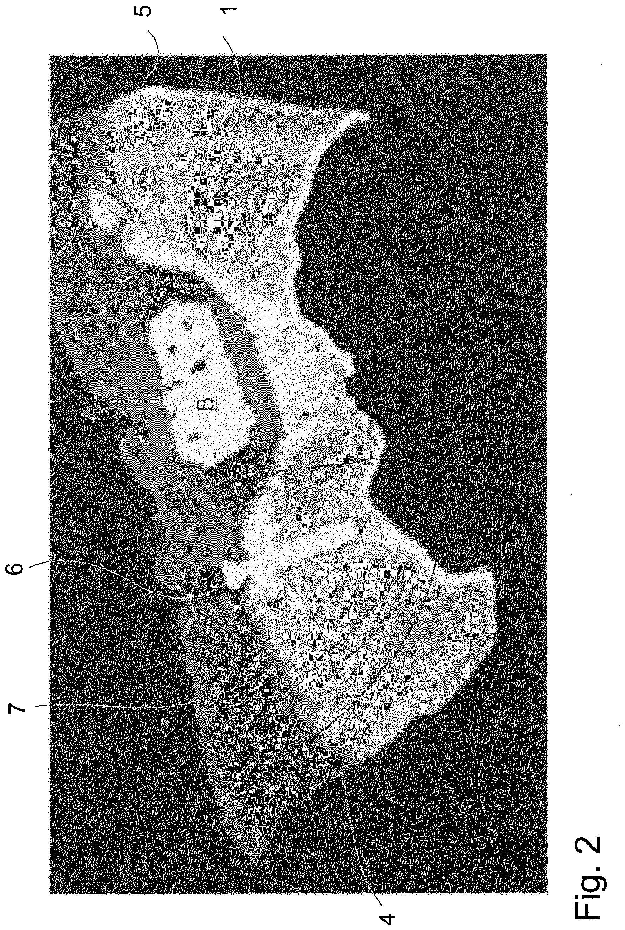

Fig. 2 zeigt die computertomographische Auswertung des oben beschriebenen Beispielversuchs.

Erkennbar und durch Einkreisung in Fig. 2 links gekennzeichnet ist die Implantationsstellen des Formteils A 1 an den Schädelkalotten 5 eines Schweines. Formteil B 1 ist in Fig. 2 auf der rechten Seite gezeigt. Zur Befestigung der Formteile 1 an der Schädelkalotte 5 wurden in diese durchgehende Bohrungen 4 eingebracht. Diese Bohrungen 4 dienten dem Durchgriff von im CT-Bild noch erkennbaren Schrauben 6, über die die Formteile 1 mit der Schädelkalotte 5 verschraubt wurden.

Fig. 2 stellt den Befund nach 8 Wochen Versuchsdauer dar. An der Stelle, an der Formteil A 1 implantiert worden war, hat der Abbau des Materials des Formteils 1 und dessen Resorption begonnen. Gleichzeitig setzte dort nachweisbar Knochenregeneration bzw. Knochenneubildung ein. Es zeigen sich gute Einheilung und vertikaler Höhengewinn. Die Schrauben 6 waren im gebildeten Knochen 7 von Knochenmaterial umgeben.

An den Stellen, an denen das Formteil B 1 implantiert worden war kam es während des Versuchszeitraums zu verringertem bis ausbleibendem Abbau des Materials. Auch konnte dort keine Knochenregeneration bzw. Knochenneubildung nachgewiesen werden. Es traten partiell Wundheilungsstörungen auf.

## Patentansprüche

1. Biokompatibles, formstabiles Formteil (1) zur Unterstützung der Neubildung eines Kieferknochens oder Kieferknochenabschnittes in einem Säugetier, bevorzugt einem Menschen, wobei das Formteil (1) auf dem Kieferknochen auflegbarer und als vom menschlichen oder tierischen Körper vollständig resorbierbarer Vollkörper ausgebildet ist, das Formteil (1) block- oder quaderförmig oder in seiner Form an eine Ausnehmung im Kieferknochen oder Kieferknochenabschnitt anpassbar ausgebildet ist, und das Formteil (1) gebildet ist aus einer Zusammensetzung bestehend aus wenigstens einem strukturgebenden Material ausgewählt ist aus der Gruppe bestehend aus Alabastergips, Hartgips, Superhartgips oder Mischungen daraus und einem porösen Granulat, gebildet aus einem Grundmaterial, das ausgewählt ist aus der Gruppe bestehend aus: Aragonit, Muschelschale, allogenem Knochenmaterial, autogenem Knochenmaterial, xenogenem Knochenmaterial, FDBA (freeze-dried bone allocrafts), DFBA (decalzified freeze-dried bone allocrafts) Algen oder Algenextrakt, Keramik, Calciumphosphat, insbesondere Tri- oder Tetracalciumphosphat, X- oder β-Tricalciumphosphat, Hydroxyl-Apatit, Calciumphosphat-Keramik, Bioglas, Knochenersatzmaterial auf Aragonitbasis (z.B. BioCoral ^{®}) oder Mischungen daraus, wobei in der Zusammensetzung das strukturgebende Material und das poröse Granulat in einem Verhältnis von zwischen 1:1 und 1:4 vorgesehen sind und das poröse Granulat eine Korngröße von zwischen 1 und 3 mm, insbesondere von zwischen 1,1 und 2 mm, bevorzugt 1,5 mm aufweist und das Formteil Kanten mit einer Kantenlänge von jeweils zwischen 1 und 5, bevorzugt von zwischen 1,5 und 3 cm aufweist.

2. Biokompatibles Formteil (1) nach Anspruch 1, **dadurch gekennzeichnet, dass** die Zusammensetzung wenigstens eine weitere Substanz beinhaltet, insbesondere wobei die wenigstens eine weitere Substanz ausgewählt ist aus der Gruppe bestehend aus Statin, Vitamin, Spurenelement, Antibiotikum, Hyaluronsäure, Hyaluronsäure-Derivat, Collagen und/oder Mischungen daraus, wobei die wenigstens eine weitere Substanz insbesondere einen Anteil von zwischen 0,1 - 3%, insbesondere von zwischen 0,2 - 1,5%, bevorzugt 0,25 % an der Zusammensetzung aufweist.

3. Biokompatibles Formteil (1) nach einem der vorhergehenden Ansprüche, **dadurch gekennzeichnet, dass** das Grundmaterial des Granulates besteht aus:
- Aragonit und
- 0-50%, insbesondere 15-35%, bevorzugt 25% Knochenmaterial, insbesondere allogenem oder autogenem Knochenmaterial.

4. Biokompatibles Formteil (1) nach einem der vorhergehenden Ansprüche, **dadurch gekennzeichnet, dass** zwischen dem Formteil (1) und dem Kieferknochen bzw. der Knochenunterlage ein Dichtungsmaterial vorgesehen ist, insbesondere wobei das Dichtungsmaterial aus einem Collagen, insbesondere Collagen Typ 1 oder einer Mischung aus Collagen Typ 1 und Collagen Typ 3 und Hyaluronsäure oder Hyaluronsäure-Derivat gebildet ist.

5. Biokompatibles Formteil (1) nach einem der vorhergehenden Ansprüche, **dadurch gekennzeichnet, dass** in dem Formteil (1) wenigstens eine Bohrung (4) für den Durchgriff von Befestigungsmitteln, insbesondere von Schrauben (6) vorgesehen ist.

6. Zusammensetzung zur Herstellung eines biokompatiblen Formteils (1) gemäß einem der Ansprüche 1 bis 5, umfassend oder bestehend aus einem strukturgebenden Material und einem Granulat, wobei das strukturgebenden Material insbesondere ausgewählt ist aus der Gruppe bestehend aus Alabastergips, Hartgips, Superhartgips oder Mischungen daraus, bevorzugt Alabastergips und/oder das Granulat aus einem Grundmaterial gebildet ist das ausgewählt ist aus der Gruppe bestehend aus: Aragonit, Muschelschale, allogenem Knochenmaterial, autogenem Knochenmaterial, xenogenem Knochenmaterial, FDBA (freeze-dried bone allocrafts), DFBA (decalzified freezedried bone allocrafts) Algen oder Algenextrakt, Keramik, Calciumphosphat, insbesondere Tri- oder Tetracalciumphosphat, X- oder β-Tricalciumphosphat, Hydroxyl-Apatit, Calciumphosphat-Keramik, Bioglas, Knochenersatzmaterial auf Aragonitbasis (z.B. BioCoral ^{®}) oder Mischungen daraus, wobei das Granulat oder das Grundmaterial bevorzugt eine umhüllende Schicht aus wenigstens einem Collagen, Hyaluronsäure und/oder Hyaluronsäure-Derivat oder Mischungen daraus aufweist, wobei das Granulat insbesondere eine Korngröße von zwischen 1 und 3 mm, insbesondere von zwischen 1,1 und 2 mm, bevorzugt 1,5 mm aufweist und/oder in der Zusammensetzung das strukturgebende Material und das Granulat bevorzugt in einem Verhältnis von zwischen 1:1 und 1:4, bevorzugt von 1:2 vorgesehen sind.

7. Zusammensetzung nach Anspruch 6, **dadurch gekennzeichnet, dass** die Zusammensetzung wenigstens eine weitere Substanz beinhaltet, insbesondere wobei die wenigstens eine weitere Substanz ausgewählt ist aus der Gruppe bestehend aus Statin, Vitamin, Spurenelement, Antibiotikum oder Mischungen daraus und wobei die wenigstens eine weitere Substanz bevorzugt einen Anteil von zwischen 0,1 - 3%, insbesondere von zwischen 0,2 - 1,5%, bevorzugt 0,25 % an der Zusammensetzung aufweist, wobei die Zusammensetzung insbesondere durch eine vollständige Resorbierbarkeit der Komponenten durch den menschlichen oder tierischen Körper gekennzeichnet ist.

8. Zusammensetzung nach einem der Ansprüche 6 oder 7, **dadurch gekennzeichnet, dass** das Grundmaterial des Granulates besteht aus:
- Aragonit und
- 0-50%, insbesondere 15-35%, bevorzugt 25% Knochenmaterial, insbesondere allogenem oder autogenem Knochenmaterial.

9. Verfahren zur Herstellung eines biokompatiblen Formteils (1), gemäß einem der Ansprüche 1 bis 8, umfassend die Schritte:
i. Herstellen einer formbaren Modelliermasse durch Anmischen der Zusammensetzung wie in Anspruch 6 definiert mit Wasser, bevorzugt destilliertem Wasser,
ii. Modellieren des Formteils (1) als block- oder quaderförmiges Formteil (1) und
iii. Trocknen des Formteils (1).

10. Verfahren nach Anspruch 9, alternativ umfassend die Schritte:
ia. Ermitteln der Form des Kieferknochens oder Kieferknochenabschnittes in oder an der das Formteil (1) an oder eingebracht werden soll, und
iia. Modellieren des Formteils (1) anhand der ermittelten Ausformung, insbesondere optional umfassend den Schritt:
ib. Erstellen eines Negativmodells der des Kieferknochens oder Kieferknochenabschnittes und/oder bevorzugt weiter umfassend den Schritt
iv. Sterilisieren des Formteils (1), insbesondere durch Bestrahlung mit Gammastrahlung wobei dass das Modellieren des Formteils (1) bevorzugt unter Verwendung einer Form erfolgt.

11. Verfahren nach einem der Ansprüche 9 oder 10, **dadurch gekennzeichnet, dass** vor oder nach dem Trockenen wenigstens eine durchgehende Bohrung (4) in das Formteil (1) eingebracht wird.

12. Biokompatiblen Formteils (1) gemäß einem der Ansprüche 1 bis 5 zur Verwendung in einem Verfahren zur Unterstützung einer Knochenneubildung im Kieferknochen, wobei das biokompatible Formteil (1) eine Grundstruktur für die Knochenneubildung zur Verfügung stellt und im Zuge der Knochenneubildung vollständig resorbiert wird, insbesondere zur Knochenaugmentation einer vorhandenen Knochenstruktur, wobei bevorzugt eine Längen-, Breiten- und/oder Höhenanpassung des Formteils (1) vor Verwendung vorgesehen ist.

13. Kit mit mehreren Formteile (1) gemäß einem der Ansprüche 1 bis 5, zur Verwendung in einem Verfahren zur Unterstützung einer Knochenneubildung im Kieferknochen, wobei die Formteile insbesondere gleiche oder unterschiedliche Größen und/oder Ausformungen aufweisen oder eine Längen-, Breiten- und/oder Höhenanpassung der Formteile (1) vor Verwendung vorgesehen ist.

## Claims

1. Biocompatible molded part (1) for supporting the formation of a new jaw bone or jaw bone section in a mammal, preferably a human, wherein the molded part (1) can be placed on the jaw bone and is designed as a solid body that can be completely resorbed by the human or animal body, the shaped part (1) is block-shaped or cuboid-shaped or is designed to be adaptable in its shape to a recess in the jawbone or jawbone section, and the shaped part (1) is formed from a composition consisting of at least one structure-giving material selected from the group consisting of alabaster gypsum, hard gypsum, superhard gypsum or mixtures thereof and a porous granulate formed from a base material selected from the group consisting of: Aragonite, Musselshell, Allogenic bone material, Autogenic bone material, Xenogenic bone material, FDBA (freeze-dried bone allocrafts), DFBA (decalzified freeze-dried bone allocrafts) algae or algae extract, Ceramic, calcium phosphate, especially tri- or tetracalcium phosphate, X- or β-tricakium phosphate, hydroxyl apatite, calcium phosphate ceramic, bioglass, aragonite-based bone substitute (e.g.B. BioCoral ^{®}) or mixtures thereof, wherein in the composition the structuring material and the porous granules are provided in a ratio of between 1:1 and 1:4 and the porous granules have a grain size of between 1 and 3 mm, in particular of between 1.1 and 2 mm, preferably 1.5 mm, and the molded part has edges with an edge length of in each case between 1 and 5, preferably of between 1.5 and 3 cm.

2. Biocompatible molded part (1) according to claim 1, **characterized in that** the composition comprises at least one further substance, in particular wherein the at least one further substance is selected from the group consisting of statin, vitamin, trace element, antibiotic, hyaluronic acid, hyaluronic acid derivative, collagen and/or mixtures thereof, wherein the at least one further substance in particular has a proportion of between 0.1 - 3%, in particular of between 0.2 - 1.5%, preferably 0.25% of the composition.

3. Biocompatible molded part (1) according to any one of the preceding claims, **characterized in that** the base material of the granules consists of:
- Aragonite and
- 0-50%, especially 15-35%, preferably 25% bone material, in particular allogeneic or autogenous bone material.

4. Biocompatible molded part (1) according to one of the preceding claims, **characterized in that** a sealing material is provided between the molded part (1) and the jawbone or the bone base, in particular wherein the sealing material is formed from a collagen, in particular collagen type 1 or a mixture of collagen type 1 and collagen type 3 and hyaluronic acid or hyaluronic acid derivative.

5. Biocompatible molded part (1) according to one of the preceding claims, **characterized in that** at least one bore (4) is provided in the molded part (1) for the passage of fastening means, in particular screws (6).

6. Composition for the production of a biocompatible molded part (1) according to any one of claims 1 to 5, comprising or consisting of a structuring material and a granulate, wherein the structuring material is in particular selected from the group consisting of alabaster gypsum, hard gypsum, superhard gypsum or mixtures thereof, preferably alabaster gypsum, and/or the granulate is formed from a base material selected from the group consisting of: Aragonite, mussel shell, allogenic bone material, autogenic bone material, xenogenic bone material, FDBA (freeze-dried bone allocrafts), DFBA (decalzified freezedried bone allocrafts) algae or algae extract, Ceramic, calcium phosphate, especially tri- or tetracalcium phosphate, X- or β-tricalcium phosphate, hydroxyl apatite, calcium phosphate ceramic, bioglass, aragonite-based bone substitute (e.g.B. BioCoral ^{®}) or mixtures thereof, wherein the granules or the base material preferably have an enveloping layer of at least one collagen, hyaluronic acid and/or hyaluronic acid derivative or mixtures thereof, wherein the granules in particular have a grain size of between 1 and 3 mm, in particular of between 1.1 and 2 mm, preferably 1.5 mm, and/or in the composition the structuring material and the granules are preferably provided in a ratio of between 1:1 and 1:4, preferably of 1:2.

7. Composition according to claim 6, **characterized in that** the composition comprises at least one further substance, in particular wherein the at least one further substance is selected from the group consisting of statin, vitamin, trace element, antibiotic or mixtures thereof and wherein the at least one further substance preferably has a proportion of between 0.1 - 3%, in particular of between 0.2 - 1.5%, preferably 0.25% of the composition, the composition being **characterized in** particular by a complete resorbability of the components by the human or animal body.

8. Composition according to any one of claims 6 or 7, **characterized in that** the base material of the granules consists of:
- Aragonite and
- 0-50%, especially 15-35%, preferably 25% bone material, in particular allogeneic or autogenous bone material.

9. Method of manufacturing a biocompatible molded article (1), according to any one of claims 1 to 8, comprising the steps of:
i. Preparing a moldable modeling composition by mixing the composition as defined in claim 6 with water, preferably distilled water,
ii. Modeling the molded part (1) as a block-shaped or cuboid-shaped molded part (1) and
iii. Drying of the molded part (1).

10. Method according to claim 9, alternatively comprising the steps of:
ia. Determining the shape of the jawbone or jawbone section in or on which the shaped part (1) is to be placed, and
iia. Modeling the molded part (1) on the basis of the determined shaping, in particular optionally comprising the step:
ib. Creation of a negative model of the jaw bone or jaw bone section and/or Prefers further comprehensive the step
iv. Sterilizing the molded part (1), in particular by irradiation with gamma radiation, wherein the modeling of the molded part (1) is preferably carried out using a mold.

11. Method according to one of claims 9 or 10, **characterized in that** at least one through-hole (4) is made in the molded part (1) before or after drying.

12. Biocompatible molded part (1) according to any one of claims 1 to 5 for use in a method for supporting new bone formation in the jaw bone, wherein the biocompatible molded part (1) provides a basic structure for the new bone formation and is completely resorbed in the course of the new bone formation, in particular for bone augmentation of an existing bone structure, wherein preferably a length, width and/or height adjustment of the molded part (1) is provided before use.

13. Kit comprising a plurality of molds (1) according to any one of claims 1 to 5, for use in a method for supporting new bone formation in the jawbone, wherein the moldings in particular have identical or different sizes and/or shapes or a length, width and/or height adjustment of the moldings (1) is provided prior to use.

## Revendications

1. Pièce moulée biocompatible (1) destinée à favoriser la formation d'un nouvel os de la mâchoire ou d'une nouvelle section d'os de la mâchoire chez un mammifère, de préférence un être humain, dans laquelle la pièce moulée (1) peut être placée sur l'os de la mâchoire et est conçue comme un corps solide qui peut être entièrement résorbé par le corps humain ou animal, la pièce moulée (1) a la forme d'un bloc ou d'un parallélépipède ou est conçue pour que sa forme puisse être adaptée à un évidement de l'os de la mâchoire ou de la section d'os de la mâchoire et la pièce façonnée (1) est formée à partir d'une composition constituée d'au moins un matériau structurant choisi dans le groupe constitué par le gypse albâtre, le gypse dur, le gypse superdur ou leurs mélanges et d'un granulé poreux formé à partir d'un matériau de base choisi dans le groupe constitué par : Aragonite, coquille de moule, matériau osseux allogène, matériau osseux autogène, matériau osseux xénogène, FDBA (allogreffes osseuses lyophilisées), DFBA (allogreffes osseuses lyophilisées décalcifiées), algues ou extrait d'algues, céramique, phosphate de calcium, en particulier phosphate tricalcique ou tétracalcique, phosphate tricalcique X ou β, hydroxylapatite, céramique de phosphate de calcium, bioglass, substitut osseux à base d'aragonite (par ex.B. BioCoral ^{®}) ou des mélanges de ceuxci, le matériau structurant et le granulat poreux étant prévus dans la composition dans un rapport compris entre 1:1 et 1:4 et le granulat poreux présentant une granulométrie comprise entre 1 et 3 mm, en particulier entre 1,1 et 2 mm, de préférence 1,5 mm, et la pièce moulée présentant des bords avec une longueur de bord respectivement comprise entre 1 et 5, de préférence entre 1,5 et 3 cm.

2. Pièce moulée biocompatible (1) selon la revendication 1, **caractérisée en ce que** la composition comprend au moins une autre substance, en particulier dans laquelle l'au moins une autre substance est choisie dans le groupe constitué par les statines, les vitamines, les oligo-éléments, les antibiotiques, l'acide hyaluronique, les dérivés de l'acide hyaluronique, le collagène et/ou leurs mélanges, dans laquelle l'au moins une autre substance a une proportion comprise entre 0,1 et 3 %, en particulier entre 0,2 et 1,5 %, de préférence 0,25 % de la composition.

3. Pièce moulée biocompatible (1) selon l'une quelconque des revendications précédentes, **caractérisée en ce que** le matériau de base des granulés est constitué de :
- Aragonite et
- 0-50%, notamment 15-35%, de préférence 25% de matière osseuse, en particulier de la matière osseuse allogène ou autogène.

4. Pièce moulée biocompatible (1) selon l'une des revendications précédentes, **caractérisée en ce qu'**un matériau d'étanchéité est prévu entre la pièce moulée (1) et le maxillaire ou la base osseuse, en particulier dans laquelle le matériau d'étanchéité est formé d'un collagène, en particulier d'un collagène de type 1 ou d'un mélange de collagène de type 1 et de collagène de type 3 et d'acide hyaluronique ou d'un dérivé d'acide hyaluronique.

5. Pièce moulée biocompatible (1) selon l'une des revendications précédentes, **caractérisée en ce qu'**au moins un alésage (4) est prévu dans la pièce moulée (1) pour le passage de moyens de fixation, notamment de vis (6).

6. Composition pour la fabrication d'une pièce moulée biocompatible (1) selon l'une quelconque des revendications 1 à 5, comprenant ou constituée d'un matériau structurant et d'un granulat, dans laquelle le matériau structurant est en particulier choisi dans le groupe constitué par le gypse albâtre, le gypse dur, le gypse superdur ou leurs mélanges, de préférence le gypse albâtre, et/ou le granulat est formé d'un matériau de base choisi dans le groupe constitué par : Aragonite, coquille de moule, matériau osseux allogène, matériau osseux autogène, matériau osseux xénogène, FDBA (allogreffes osseuses lyophilisées), DFBA (allogreffes osseuses lyophilisées décalcifiées), algues ou extrait d'algues, céramique, phosphate de calcium, en particulier phosphate tricalcique ou tétracalcique, phosphate tricalcique X ou β, hydroxylapatite, céramique de phosphate de calcium, bioglass, substitut osseux à base d'aragonite (par exemple B. BioCoral ^{®}) ou leurs mélanges, les granulés ou le matériau de base présentant de préférence une couche d'enrobage constituée d'au moins un collagène, d'acide hyaluronique et/ou d'un dérivé d'acide hyaluronique ou leurs mélanges, les granulés présentant en particulier une granulométrie comprise entre 1 et 3 mm, notamment entre 1,1 et 2 mm, de préférence 1,5 mm, et/ou le matériau structurant et les granulés étant de préférence présents dans la composition dans un rapport compris entre 1:1 et 1:4, de préférence 1:2.

7. Composition selon la revendication 6, **caractérisée en ce que** la composition comprend au moins une autre substance, en particulier dans laquelle l'au moins une autre substance est choisie dans le groupe constitué d'une statine, d'une vitamine, d'un oligo-élément, d'un antibiotique ou de leurs mélanges et dans laquelle l'au moins une autre substance a de préférence une proportion comprise entre 0,1 et 3 %, en particulier entre 0,2 et 1,5 %, de préférence 0,25 % de la composition, la composition étant **caractérisée** en particulier par une résorbabilité complète des composants par le corps humain ou animal.

8. Composition selon l'une quelconque des revendications 6 ou 7, **caractérisée en ce que** le matériau de base des granulés est constitué de :
- Aragonite et
- 0-50%, notamment 15-35%, de préférence 25% de matière osseuse, en particulier de la matière osseuse allogène ou autogène.

9. Procédé de fabrication d'une pièce moulé biocompatible (1), selon l'une quelconque des revendications 1 à 8, comprenant les étapes suivantes :
i. Préparation d'une composition de modelage moulable en mélangeant la composition telle que définie dans la revendication 6 avec de l'eau, de préférence de l'eau distillée,
ii. Modélisation de la pièce moulée (1) sous la forme d'une pièce moulée (1) en forme de bloc ou de cube et
iii. Séchage de la pièce moulée (1).

10. Procédé selon la revendication 9, comprenant alternativement les étapes suivantes :
ia. Déterminer la forme de la mâchoire ou de la section de la mâchoire dans ou sur laquelle la pièce façonnée (1) doit être placée, et
iia. Modélisation de la pièce moulée (1) sur la base de la mise en forme déterminée, comprenant en particulier éventuellement l'étape :
ib. Création d'un modèle négatif de l'os de la mâchoire ou de la section de l'os de la mâchoire et/ou Préfère approfondir l'étape
iv. Stérilisation de la pièce moulée (1), en particulier par irradiation avec un rayonnement gamma, le modelage de la pièce moulée (1) étant de préférence réalisé à l'aide d'un moule.

11. Procédé selon l'une des revendications 9 ou 10, **caractérisé en ce qu'au** moins un trou traversant (4) est réalisé dans la pièce moulée (1) avant ou après le séchage.

12. Pièce moulée biocompatible (1) selon l'une quelconque des revendications 1 à 5, destinée à être utilisée dans un procédé pour soutenir la formation d'un nouvel os dans l'os de la mâchoire, dans laquelle la pièce moulée biocompatible (1) fournit une structure de base pour la formation du nouvel os et est complètement résorbée au cours de la formation du nouvel os, en particulier pour l'augmentation osseuse d'une structure osseuse existante, dans laquelle de préférence un ajustement en longueur, en largeur et/ou en hauteur de la pièce moulée (1) est prévu avant l'utilisation.

13. Kit comprenant une pluralité de moules (1) selon l'une quelconque des revendications 1 à 5, destiné à être utilisé dans un procédé de soutien de la néoformation osseuse dans l'os de la mâchoire, dans lequel les moules ont notamment des tailles et/ou des formes identiques ou différentes ou un ajustement en longueur, en largeur et/ou en hauteur des moules (1) est prévu avant l'utilisation.
